# EUROPÄISCHE PATENTSCHRIFT

(11) **EP 4 251 431 B1**
(45) Veröffentlichungstag und Bekanntmachung des Hinweises auf die Patenterteilung: **23.09.2026**
(21) Anmeldenummer: 21820134.1
(22) Anmeldetag: 23.11.2021
(51) Int. Cl.: B42D 25/378, B41M 3/14

(54) **METHODE ZUR KENNZEICHNUNG VON PRODUKTEN MIT OPTISCHEM SICHERHEITSMERKMAL MIT ZEITLICHER DIMENSION**
METHOD FOR LABELLING PRODUCTS WITH AN OPTICAL SECURITY FEATURE WITH A TEMPORAL DIMENSION
PROCÉDÉ PERMETTANT DE MARQUER DES PRODUITS AYANT UNE CARACTÉRISTIQUE DE SÉCURITÉ OPTIQUE AVEC UNE DIMENSION TEMPORELLE

(30) Priorität: 26.11.2020 DE 102020131382
(43) Veröffentlichungstag der Anmeldung: 04.10.2023
(73) Patentinhaber: Fraunhofer-Gesellschaft zur Förderung der angewandten Forschung e.V., 80686 München (DE)
(72) Erfinder: JOCHUM, Tobias, 20146 Hamburg (DE); NIEHAUS, Jan, 20146 Hamburg (DE)
(74) Vertreter: Maiwald GmbH
(86) Internationale Anmeldenummer: PCT/EP2021/082595
(87) Internationale Veröffentlichungsnummer: WO 2022/112209

(56) Entgegenhaltungen:
- EP-A1- 3 301 655
- WO-A1-2013/188927
- DE-A1- 102015 219 400
- DE-A1- 102016 011 180
- JP-A- 2020 002 030
- US-A1- 2013 161 396
- US-B1- 9 382 432

## Beschreibung

Die vorliegende Erfindung beruht auf einer Methode zur Kennzeichnung von Produkten mit Hilfe von zwei oder mehreren Tintenformulierungen, die jeweils ein oder mehrere Photolumineszenzfarbstoffe enthalten, die unter Photonenanregung eine Strahlung im Bereich von 380-3000 nm emittieren und sich durch verschiedene Photolumineszenzlebensdauern unterscheiden, zur Abspeicherung von Informationen, in Serialisierungs- und/oder Track & Tracesystemen und zur Dokumentensicherheit.

Die vorliegende Erfindung betrifft ebenfalls ein optisches, zeitabhängiges Sicherheitsmerkmal, seine Verwendung, ein Serialisierungs- und/oder Track and Tracesystem und die Verwendung eines auf ein Produkt aufgedruckten Codes.

### Technischer Hintergrund

Produktfälschungen verursachen einen weltweiten wirtschaftlichen Schaden von mehreren hundert Milliarden US-Dollar. Alleine in Europa entsteht durch Produktfälschungen ein wirtschaftlicher Schaden von mehr als 80 Milliarden Euro. Die Bandbreite der gefälschten Produkte ist immens. Es werden vermehrt Kosmetika, Uhren, Tabak und medizinische Produkte gefälscht. Die Pharma- und Tabakindustrie - bedingt durch EU-Vorgaben (2011/62/EU und 2014/40 EU) - haben im Jahr 2019 ein Serialisierungssystem zur Überwachung ihrer Produkte eingeführt. Dabei wird jede Produktverpackung mit einem speziellen, einzigartigen Code ausgestattet und dieser in eine zentrale Datenbank abgespeichert. Dabei einstehen mehrere problematische Szenarien:
- Hacken der Datenbank. Hierzu muss beachtet werden, dass kein IT-System allumfassend gegen Hacker-Angriffe von Dritten abgesichert werden kann. Die Hacker können entweder ihre eigenen Codes in der zentralen Datenbank abspeichern/hinzufügen oder die einzigartigen Codes der anderen Firmen abpassen. Somit kann nicht mehr verifiziert werden, welches Produkt eine Fälschung und welches Produkt das Original ist.
- Weitergabe der Codes an Dritte. Die einzigartigen Codes können durch Personal an Dritte weitergegeben werden. Diese können dann die Codes auf die gefälschten Produkte drucken, so dass diese laut Datenbank als "echt" anzusehen sind.
- Die Übertragung der Codes auf eine andere Verpackung. Sobald der Code von der Originalverpackung auf die Verpackung des Duplikats übertragen und die Originalverpackung entsorgt wird, können gefälschte Präparate als Originalprodukte verkauft werden. Dieser Betrug ist schwer nachzuvollziehen, denn das Datenbanksystem bestätigt, dass das vorliegende Produkt keine Fälschung ist. Dieses Risikoszenario wäre z.B. denkbar beim Umverpacken von gestohlenen Produkten/Medikamenten und bei illegalem Handel mit Produkten im Internet oder mit geschmuggelter Ware.

Aufgrund von diesen drei kritischen Punkten ist es sehr wichtig, ein "cyber-physical system (CPS)" zu entwickelten/entwerfen; also eine Kombination aus digitalen und physikalischen Merkmalen.

Auch für Anwendung in der Industrie 4.0 und Logistik 4.0 (Lieferkettensicherheit) werden CPS benötigt. Bei der Industrie 4.0 soll jeder Arbeitsprozess digitalisiert und vernetzt werden. Maschinen werden die jeweiligen Arbeiten von Morgen übernehmen. Das ist nur möglich, wenn ein hohes Maß an Sicherheit gewährleistet werden kann. Maschinen müssen Maschinen vertrauen können. Dies schafft man, indem jedes Endprodukt (auch Teilprodukte, Werkzeuge, Prozesse...) eines Arbeitsschritts mithilfe einer Identität (à la Track & Trace) ausgestattet ist. Allerdings gibt es Unterschiede bei Identitäten. Man unterscheidet 3 Typen von Identitäten:
- Identität (ID): z.B.: QR-Code
- Eindeutige Identität: z.B.: QR-Code mit Seriennummer
- Sichere Identität: z.B. QR-Code mit zweitem Faktor

Bezüglich Fälschungssicherheit von Produkten wurden in den letzten Jahren hauptsächlich an zwei konkurrierenden Lösungen gearbeitet, nämlich Track & Trace und Authentifizierungslösungen, speziell auf optischer Basis.

Track & Trace Programme (US 9,027,147; US 8,898,007; US 2009/0096871; US 8,700,501) werden genützt, um die eindeutige Verfolgung (Track) und Rückverfolgung (Trace) aller Prozessschritte in der Produktions- und Lieferkette zu gewährleisten. Außerdem ermöglichen sie umfassende Kontrollmöglichkeiten für den Hersteller und Transparenz für den Verbraucher, da Standorte und Wege von Produkten und Dokumenten lückenlos dokumentiert werden können. Hierfür werden Informationsspeicher benötigt. Je nach Informationsspeicher unterscheidet man einen 1-dimensionalen Barcode (Strichpunkt), 2-dimensionalen Barcode (QR-Code), 3-dimensionalen Barcode (Farbiger Barcode EP 2100277B1) und den 4-dimensionalen Barcode (Farbiger Barcode + Zeitliche Komponente US 2013/0161395 A1). Bei dem derzeit etablierten 4D-Barcode wird ein farbiges Muster zeitlich verändert. Diese Art ist jedoch nicht druckbar, da die zeitliche Veränderung ein Display mit Speichereinheit erfordert. Sie ist daher nicht auf Produktverpackungen einzusetzen. Für Authentifizierungslösungen ist das Zusammenspiel von Fälschungssicherheit und Design elementar wichtig. Es werden zum Teil hochdekorative und innovative Authentifizierungslösungen verwendet, um den Konsumenten vor Manipulation zu schützen. Dazu gehören Authentifizierungslösungen, welche für das menschliche Auge sowohl sichtbar als auch unsichtbar sind. Ebenfalls zur Gattung der Authentifizierungslösungen gehören Hologramme und irisierende-Materialen. Des Weiteren unterscheidet man in Bezug auf Authentifizierungslösungen drei unterschiedliche Typen: sichtbare (overt), unsichtbare (covert) und forensische Authentifizierungslösungen. Ein sichtbares Security-Label ist z.B. ein Hologramm. Ein unsichtbares Security-Label ist z.B. eine Geheimtinte und ein forensisches Security-Label ist eine Markersystem, welche komplexe Detektionsgeräte (z.B. Mikroskope) benötigen. Ein Abspeichern von Information ist bei dieser Technologie nach jetzigem Stand nicht möglich. Dies ist ein entscheidender Nachteil gegenüber den Track & Trace Lösungen.

US 9,382,432 B1, WO 2013/188927 A1 und US 6,692,031 B1 beschreiben unsichtbare Authentifzierungslösungen, die auf Basis einer Art von Geheimtinte beruht, die verschiedene Quantendots mit unterschiedlichen Fluoreszenzlebensdauern enthält. Durch die unterschiedlichen Fluoreszenzdauern verändert sich das Fluoreszenzspektrum der Geheimtinte über die Zeit, so dass diese eine zeitliche Dimension erhält.

Beide Technologien werden aktuell kaum zu einer CPS kombiniert. DE 10 2019 216 003.4 beschreibt eine Methode zur Kennzeichnung von Produkten mit Hilfe einer Tintenformulierung, die halbleitende anorganische Nanokristalle enthält, die unter Photonenanregung eine Strahlung im Bereich von 750-1800 nm emittieren, in Serialisierungs- und/oder Track & Tracesystemen.

DE 10 2015 219 400 A1 offenbart ein Verfahren zur Prüfung der Identität und/oder Echtheit eines Gegenstandes, bei dem ein Identifikationsmerkmal mit einer aus Pigmenten eines Farbmittels oder aus Partikeln von mindestens einem Farbstoff ausgebildeten Zufallsstruktur verwendet wird.

EP 3 301 655 A2 offenbart ein Sicherheitsmerkmal, das eine Mehrzahl von lumineszierenden Flächenelementen aufweist, wobei die Lumineszenzlebensdauer zwischen den Flächenelementen variiert.

DE 10 2016 011 180 A1 offenbart ein Wertdokument mit einer Sicherheitsmarkierung in Form von mindestens zwei lumineszierenden Substanzen, die einem definierten relativen Mengenanteil vorliegen und durch einen Anregungspuls gemeinsam anregbar sind. Die Zeitverläufe der Intensitäten sind verschieden und mindestens eine lumineszierende Substanz weist einen nichtmonoexponentiellen Zeitverlauf auf.

Die vorliegende Erfindung beschreibt ein innovatives, druckfähiges CPS-Modell, in dem Daten in einem physikalischen Sicherheitsmerkmal eines mehrdimensionalen Codes abgespeichert werden, das zusätzlich eine zeitliche Dimension enthält. Somit sind die gelabelten Produkte fälschungssicherer, denn die Fälscher müssten das weiterführende physikalische Sicherheitsmerkmal und/oder das Markersystem auf das Produkt drucken. Die erfindungsgemäße Methode stellt eine sichere Identität zur Verfügung, die als CPS Anwendung in der Industrie 4.0 und Logistik 4.0 oder der Dokumentensicherung finden kann.

### Gegenstand der Erfindung

Die vorliegende Erfindung betrifft eine Methode zur Kennzeichnung von Produkten, wie es in dem Anspruch 1 definiert ist.

Ebenfalls betrifft die Erfindung ein optisches, zeitabhängiges Sicherheitsmerkmal auf mindestens einer Fläche der Oberfläche eines Produktes in Form eines mehrdimensionalen Codes, wie es in dem Anspruch 12 definiert ist.

Weiterhin betrifft die Erfindung die Verwendung des optischen, zeitabhängigen Sicherheitsmerkmals wie es in dem Anspruch 13 definiert ist.

Zudem betrifft die Erfindung ein Serialisierungs- und/oder Track & Tracesystem, das ein optisches, zeitabhängiges Sicherheitsmerkmal enthält, das einen auf ein Produkt aufgedruckten mehrdimensionalen Code gemäß Anspruch 14.

Weiterhin betrifft die Erfindung die Verwendung eines auf ein Produkt aufgedruckten mehrdimensionalen Codes gemäß Anspruch 15.

### Definitionen

Die Bezeichnung "Produkte" in Sinne der vorliegenden Erfindung umfasst die Produkte selbst, soweit kennzeichnungsfähig, deren Verpackungen, Produktschilder (Tags), Barcode-Karten und Barcode-Etiketten, sowie alle anderen Möglichkeiten, mit denen ein Produkt üblicherweise während des Produktionsprozesses und/oder des Transports gekennzeichnet würden. Produkte umfassen Erzeugnisse und deren Zwischenstufen, Handelsgüter sowie Dokumente. Einige Beispiele sind im Folgenden aufgeführt: ein Markenprodukt, ein Verbraucherprodukt, ein pharmazeutisches Produkt, ein Gesundheitsprodukt, ein Ernährungsprodukt, eine Komponente, eine Hardwarekomponente, eine elektronische Komponente, ein Computerchip, ein Buch, ein Handbuch.

Die Bezeichnung "Dokumente" im Sinne der vorliegenden Erfindung umfasst natürliche, zellulose-basierte Substrate, künstliche polymer-basierte Substrate und Mischungen aus selbigen, insbesondere Banknoten, Ausweise, Pässe, Geburtsurkunden, Fahrausweise, Eintrittskarten und andere Tickets. Einige weitere Beispiele sind im Folgenden aufgeführt: einen Scheck, eine Anleihe, eine Bankkarte, eine Kreditkarte, eine Scheckkarte, eine Währung, eine Geldkarte, einen Identifikationsgegenstand, einen Identitätsgegenstand, einen Zugangsgegenstand, einen Gegenstand zur Erteilung einer Erlaubnis, ein Personalausweis, ein Personalausweis, ein Führerschein, ein personalisierter Gegenstand, ein Reisepass, ein Dokument, ein Papierdokument, ein Sicherheitsdokument, eine Briefmarke, ein personalisiertes Dokument, ein Zertifikat, ein Aktienzertifikat, ein Schuldzertifikat, ein Vertrag, eine Versicherungspolice, ein Testament, ein Parkticket, ein Transportticket oder ein Ticket für die Zulassung zu einer Veranstaltung.

Die Bezeichnung "Tintenformulierung" in Sinne der vorliegenden Erfindung umfasst jedes beliebige Lösungsmittel sowie Kombinationen aus selbigen sowie typische Additive, die zur Herstellung einer druckfähigen Flüssigkeit geeignet sind.

Photolumineszenz bezeichnet die Emission von Photonen nach vorheriger Anregung mittels Photonen höherer Energie, meist im ultravioletten, aber auch sichtbaren Bereich. Durch die Anregung wird ein Elektron in einen höheren Energiezustand gehoben. Beim Zurückfallen in einen niedrigeren Energiezustand wird diese Energie in Form von Photonen wieder abgegeben. In einem lumineszierenden Stoff unterscheidet man grob zwei Arten der Anregung: bei der Fluoreszenz fällt das Elektron aus einem höheren Singulett-Zustand in den niedrigeren Energiezustand zurück, während bei der Phosphoreszenz das angeregte Elektron in einen erhöhten durch einen nach der Spinauswahl verbotenen Übergang in einen erhöhten Triplett-Zustand übergeht, aus dem es wiederum durch einen nach der Spinauswahl verbotenen Übergang in den niedrigeren Energiezustand zurückfällt.

Unter dem Begriff der Photolumineszenz-Lebensdauer gliedert sich die FluoreszenzLebensdauer als auch die Phosphoreszenz-Lebensdauer.

Die Bezeichnung "Fluoreszenzlebensdauer" im Sinne der vorliegenden Erfindung gibt die mittlere Zeit an, die ein Molekül bei der Fluoreszenz in einem angeregten Singulett-Zustand bleibt, bevor es ein Photon emittiert und damit in den niedrigeren Energiezustand zurückkehrt.

Die Bezeichnung "Phosphoreszenzlebensdauer" im Sinne der vorliegenden Erfindung gibt die mittlere Zeit an, die ein Molekül bei der Phosphoreszenz in einem angeregten Triplett-Zustand bleibt, bevor es ein Photon emittiert und damit in den Grundzustand zurückkehrt.

Die Bezeichnung "mehrdimensionaler Code" im Sinne der vorliegenden Erfindung umfasst mindestens eine räumliche Dimension (i.e. in x-Richtung), eine farbliche Dimension der Photolumineszenz der Photoluminszenzfarbstoffe und eine zeitliche Dimension, die auf der Messung der Photolumineszenz-Lebensdauern der verwendeten Photolumineszenzfarbstoffe beruht. Weitere mögliche Dimensionen sind eine weitere räumliche Dimension (i.e. in y-Richtung) sowie eine farbliche Dimension, über die Eigenfarbe der Farbstoffe auf einem Substrat als mehrfarbiger Code.

Die Bezeichnung "Drucken" in Sinne der vorliegenden Erfindung umfasst die Abscheidung von Pigmenten auf oder in ein festes Substrat. Typische Beispiele sind, aber nicht ausschließlich, Digitaldruck, Tintenstrahldruck, Siebdruck, Transferdruck, Stempeldruck, Rolle zu Rolle, Druck ohne Kontakt, Laserdruck sowie weitere Verfahren.

Die Bezeichnung "Bestrahlung" in Sinne der vorliegenden Erfindung umfasst die Anregung des Photolumineszenz-Emissionssignals und der Photolumineszenz-Lebensdauer. Für das Emissionssignal können verschiedene Anregungsquellen dienen. Einige Beispiele dafür sind: LEDs, Helium/Xenon-Lampen, Laserdioden. Für die Photolumineszenz-Lebensdauer wird üblicherweise der Puls der Anregung zeitlich kürzer als die Photolumineszenz-Lebensdauer der Pigmente sein. Hierfür werden gerne Laserdioden genommen.

Die Bezeichnung "Detektion" im Sinne der vorliegenden Erfindung umfasst auf der einen Seite die Erfassung des Emissionssignals der photolumineszenten Pigmente. Hierfür eignen sich Detektoren aus Silizium und Germanium. Auf der anderen Seite umfasst es aber auch die räumliche und zeitliche Erfassung der photolumineszenten Abklingzeiten der jeweiligen Pigmente. Hierfür sind spezielle Sensoren und Detektoren nötig, welche eine zeitliche Abfolge auflösen können.

### Figuren

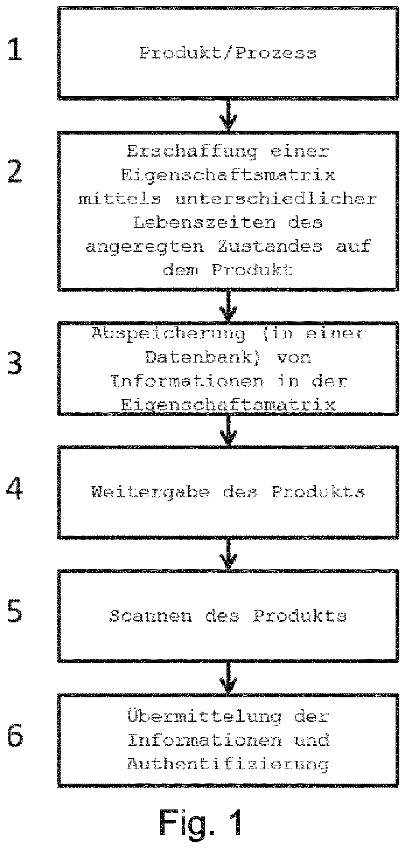

Figur 1 zeigt eine Übersicht über eine mögliche Ausführungsform der erfindungsgemäßen Methode zur Kennzeichnung von Produkten.

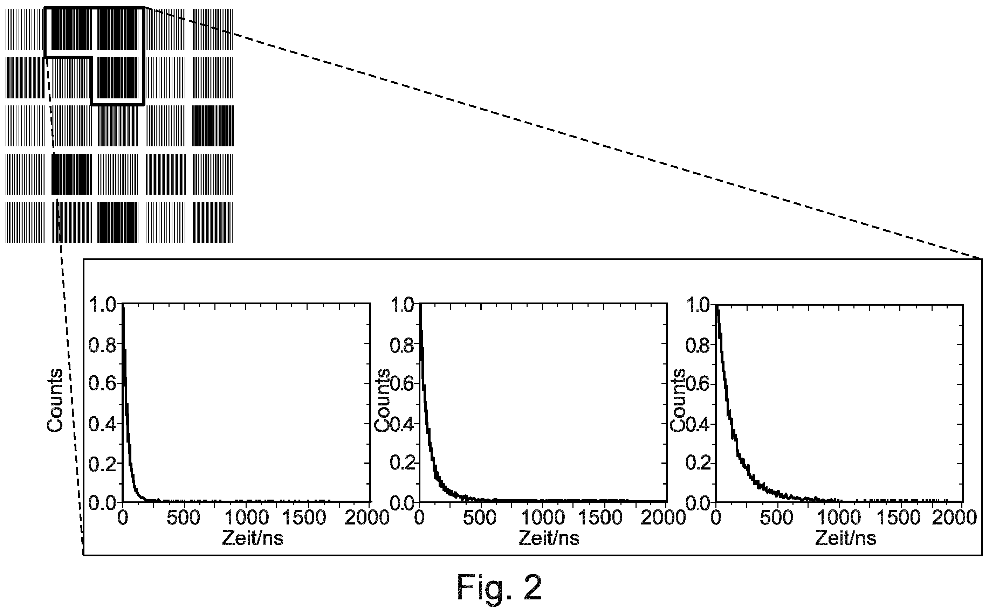

Figur 2 zeigt ein Beispiel eines vierdimensionalen Codes mit zwei räumlichen Dimensionen in x- und y-Richtung, einer farblichen Dimension durch die Fluoreszenz der Fluoreszenzfarbstoffe, dargestellt durch unterschiedliche Farben, sowie einer zeitliche Dimension durch die unterschiedlichen Fluoreszenzlebensdauern der drei "roten" Fluoreszenzfarbstoffe nach gepulster Anregung. Die Abklingzeit sind dargestellt als Counts über die Zeit in ns.

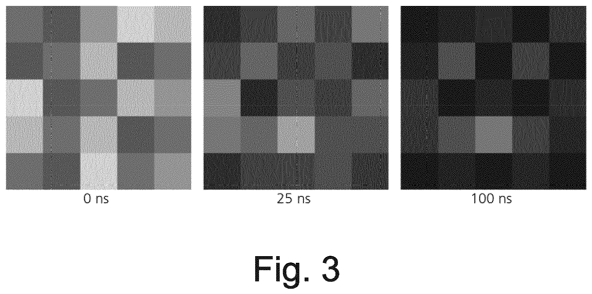

Figur 3 zeigt ein simuliertes Beispiel eines vierdimensionalen Codes mit zwei räumlichen Dimensionen in x- und y-Richtung, einer farblichen Dimension durch die Photolumineszenz der Photolumineszenzfarbstoffe, dargestellt durch unterschiedliche Farben, sowie einer zeitlichen Dimension durch die unterschiedlichen Fluoreszenzlebensdauern als detektierte Bilder in einem Zeitraum von 0 ns (links), 25 ns (Mitte) und 100 ns (rechts) nach Beginn der Bestrahlung mit Photonen.

### Detaillierte Beschreibung der Erfindung

Die vorliegende Erfindung betrifft eine Methode zur Kennzeichnung von Produkten, gemäß Anspruch 1.

Es werden zunächst zwei oder mehrere Tintenformulierung bereitgestellt, die alle jeweils ein oder mehrere Photolumineszenzfarbstoffe, bevorzugt einen Photolumineszenzfarbstoff, enthalten, die unter Photonenanregung eine Strahlung im Bereich von 380-3000 nm emittieren und eine Photolumineszenz-Lebensdauer von 1 ns - 1 min aufweisen.

Die Anzahl der Tintenformulierungen ist generell nicht begrenzt und unterliegt lediglich praktischen, ökonomischen und sicherheitsrelevanten Gesichtspunkten. Generell gilt, je größer die Anzahl der Tintenformulierungen, desto höher die Komplexität und somit der Informationsgehalt des zu druckenden einzigartigen mehrdimensionalen Codes. Die Anzahl der Tintenformulierungen ist sicherlich nach oben begrenzt durch die Höchstanzahl der von dem verwendeten Druckers verarbeitbaren Tintenformulierungen und durch preisliche Einschränkungen. Üblicherweise kommen in der erfindungsgemäßen Methode zwischen 2 und 30 Tintenformulierungen, bevorzugt 2 bis 25 Tintenformulierungen, stärker bevorzugt 2 bis 20 Tintenformulierungen, am stärksten bevorzugt 3 bis 15 Tintenformulierungen zum Einsatz.

Die Tintenformulierungen sind vorzugsweise handelsübliche Tintenformulierungen geeignet für die Abscheidung von Pigmenten auf oder in ein festes Substrat. Typische Beispiele sind, aber nicht ausschließlich, Digitaldruck, Tintenstrahldruck, Siebdruck, Transferdruck, Stempeldruck, Rolle zu Rolle, Druck ohne Kontakt, Laserdruck sowie weitere Verfahren. Diesen handelsüblichen Tintenformulierungen können dann ein oder mehrere Photolumineszenzfarbstoffe zugesetzt werden.

In einer anderen Ausführungsform enthalten die Tintenformulierungen außer den Photolumineszenzfarbstoffen keine weiteren Farbpigmente. In dieser Ausführungsform ist der mit den Tintenformulierungen aufgedruckte mehrdimensionale Code mit menschlichem Auge wegen der Konzentration der Photolumineszenzfarbstoffe nicht sichtbar. Der mehrdimensionale Code ist somit nicht sofort ersichtlich sondern kann nur nach Bestrahlung des mit den Tintenformulierungen bedruckten Produktes mit Photonen über die Detektion der von dem bestrahlten Produkts emittierten Strahlung im Bereich von 380-3000 nm entdeckt und ausgelesen werden.

In einer nächsten Ausführungsform enthalten die Tintenformulierungen außer den Photolumineszenzfarbstoffen keine weiteren Farbpigmente. In dieser Ausführungsform ist der mit den Tintenformulierungen aufgedruckte mehrdimensionale Code mit menschlichem Auge wegen der hohen Konzentration der Tintenformulierungen sichtbar (Eigenfarbe der Photolumineszenzfarbstoffe). Der mehrdimensionale Code ist somit sofort ersichtlich. Nach der Bestrahlung des mit den Tintenformulierungen bedruckten Produktes mit Photonen kann die emittierten Strahlung im Bereich von 380-3000 nm entdeckt und ausgelesen werden.

In einer vierten Ausführungsform wird zunächst ein mehrdimensionaler Code mit einer handelsüblichen Tintenformulierung auf mindestens eine Oberfläche des Produkts gedruckt. In einem zweiten Schritt werden dann die Tintenformulierungen, die die Photolumineszenzfarbstoffe enthalten, punktuell in Form von Tropfen und/oder anderen Mustern, wie beispielsweise Flächen, Streifen, Linien, geometrische Figuren, wie Kreise, Dreiecke, Rechtecke, Vielecke etc., alphanummerischen Zeichen, oder Kombinationen daraus, auf den bestehenden mehrdimensionalen Code gedruckt. In dieser Ausführungsform enthalten die Tintenformulierungen außer den Photolumineszenzfarbstoffen vorzugsweise keine Pigmente, so dass die Tropfen und/oder der weitere mehrdimensionale Code für das menschliche Auge nicht sichtbar sind.

In einer fünften Ausführungsform wird zunächst ein mehrdimensionaler Code mit einer handelsüblichen Tintenformulierung auf mindestens eine Oberfläche des Produkts gedruckt. In einem zweiten Schritt werden dann die Tintenformulierungen, die die Photolumineszenzfarbstoffe enthalten, punktuell in Form von weiteren mehrdimensionalen Codes aufgebracht. In dieser Ausführungsform enthalten die Tintenformulierungen außer den Photolumineszenzfarbstoffen vorzugsweise keine Pigmente, so dass die Tropfen und/oder der weitere mehrdimensionale Code für das menschliche Auge nicht sichtbar sind.

In einer sechsten Ausführungsform wird nach einer der vorhergegangenen Ausführungsformen ein zusätzlicher einzigartiger Code beim Druckprozess - bedingt durch z.B. die hohe Druckfrequenz und Ablenkungen der Tintentropfen - generiert.

In einer siebten Ausführungsform wird der mehrdimensionale Code nach einer der vorhergegangenen Ausführungsformen auf mindestens ein Etikett gedruckt, das anschließend auf mindestens eine Oberfläche des Produkts geklebt wird.

In einer achten Ausführungsform wird der einzigartige mehrdimensionale Code nach einer der vorhergegangenen Ausführungsformen auf Produktschilder (Tags), Barcode-Karten und/oder Barcode-Etiketten gedruckt.

In einer neunten Ausführungsform wird der einzigartige mehrdimensionale Code nach einer der vorhergegangenen Ausführungsformen auf Dokumente gedruckt.

Jede Tintenformulierung enthält einen oder mehrere, etwa 2, 3, 4, 5 oder mehr Photolumineszenzfarbstoffe. Vorzugsweise enthält jede Tintenformulierung einen Photolumineszenzfarbstoff.

Die Photolumineszenzlebensdauern der eingesetzten Photolumineszenzfarbstoffe liegen üblicherweise im Bereich von 1 ns bis 1 min, vorzugsweise im Bereich von 1 ns bis 1 s, stärker bevorzugt im Bereich von 1 ns bis 1 ms, am stärksten bevorzugt im Bereich von 5 ns bis 100 µs.

Die Photolumisenzlebensdauern der verschiedenen in den Tintenformulierungen eingesetzten Photolumineszenzfarbstoffe unterscheiden sich üblicherweise im Bereich von 1 ns bis 1 min, vorzugsweise im Bereich von 1 ns bis 1 s, stärker bevorzugt im Bereich von 1 ns bis 1 ms, am stärksten bevorzugt im Bereich von 5 ns bis 100 µs.

Die Photolumineszenzlebensdauern der Photolumineszenzfarbstoffe lassen sich beispielsweise in Abklingdiagrammen von Emissionsspektren identifizieren.

Die Photolumineszenzfarbstoffe können ausgewählt werden aus Fluoreszenzfarbstoffen, Phosphoreszenzfarbstoffen und Mischungen daraus. Fluoreszenzfarbstoffe sind Farbstoffe, die nach Photonenanregung eine Fluoreszenzstrahlung emittieren, während Phosphoreszenzfarbstoffe Farbstoffe sind, die nach Photonenanregung eine Phosphoreszenzstrahlung emittieren.

Die Auswahl der Phosphoreszenzfarbstoffe ist generell lediglich darin beschränkt unter Photonenanregung eine Strahlung im Bereich von 380-3000 nm emittieren, bevorzugt von 450 bis 1800 nm, am stärksten bevorzugt von 750 nm bis 1100 nm.

Die in diesem Patent verwendeten Phosphoreszenzfarbstoffe können unter Photonenanregung sowohl einen "Stoke-Shift", als auch einen "Anti-Stoke-Shift" aufweisen. Des Weiteren können Leuchtstoffe sowohl Fluoreszenz-, als auch Phosphoreszenz-Verhalten aufweisen. Die verwendeten Leuchtstoffe können sowohl organische, als auch anorganische Kristalle/Molekülen sein.

Fluoreszenzfarbstoffe sind üblicherweise ausgewählt aus organischen Fluoreszenzfarbstoffen und anorganischen Fluoreszenzfarbstoffen oder Mischungen daraus.

Organische Farbstoffe können aus den Klassen der Proteine und Peptide, kleinen organischen Moleküle, synthetischen Oligomere und Polymere sowie Multikomponentensystemen ausgewählt werden.

Typische Beispiele für Polymere und Peptide sind Green Fluorescent Protein (GFP), Yellow Fluorescent Protein (YFP) oder Red Fluorescent Protein (RFP). Nicht-proteinische organische Fluoreszenzfarbstoffe gehören üblicherweise den Klassen der Xanthenderivate, Cyaninderivate, Squarainderivate, Squarain-Rotaxanderivate, Naphathalenderivate, Cumarinderivate, Oxadiazolderivate, Anthrazenderivate, Pyrenderivate, Oxazinderivate, Acridinderivare, Arylmethinderivare, Tetrapyrrolderivate und Dipyrromethanderivate an. Organische Fluoreszenzfarbstoffe sind üblicherweise käuflich erwerblich in allen Emissionsspektralfarben von blau (ab 380 nm) bis rot (bis 3000 nm).

Geeignete organische Farbstoffe mit Emissionsspektralfarben ab 800 nm sind dabei beispielsweise beschrieben in EP 0 933 407, US 5,282,894, US 5,665,151, WO 1998/018871, WO 2003/038003, US 10,119,071 und US 5,542,971.

Geeignete anorganische Farbstoffe sind vorzugsweise halbleitende anorganische Nanokristalle.

Die halbleitenden anorganischen Nanokristalle sind vorzugsweise ausgewählt aus der Gruppe der Perovskite, I-VI-Halbleiter, II-VI-Halbleiter, III-V-Halbleiter, IV-VI-Halbleiter, I-III-VI-Halbleiter, Carbon dots und Mischungen daraus.

Beispiele für geeignete halbleitende anorganische Nanokristalle sind unter anderem AgS, AgSe, AgTe, CdS, CdSe, CdTe, PbS, PbSe, PbTe, SnTe, ZnS, ZnSe, ZnTe, InP, InAs, Cu₂S, In₂S₃, InSb, GaP, GaAs, GaN, InN, InGaN, ZnSSe, ZnSeTe, ZnSTe, CdSSe, CdSeTe, HgSSe, HgSeTe, HgSTe, ZnCdS, ZnCdSe, ZnCdTe, ZnHgS, ZnHgSe, ZnHgTe, CdHgS, CdHgSe, CdHgTe, ZnCdSSe, ZnHgSSe, ZnCdSeTe, ZnHgSeTe, CdHgSSe, CdHgSeTe, CdSeCdS, CuInS2, CuInSe₂, CuInGaSe₂, CuInZnS₂, CuZnSnSe₂, CuIn(S,Se)₂, CuInZn(S,Se)₂, AgIn(S,Se)₂. Weitere geeignete Beispiele, aber nicht ausschließlich, sind Perovskite-Materialien mit der allgemeinen Formel ABX₃ oder A₄BX₆, wobei X ausgewählt aus Cl, Br, I, O und/oder Mischungen daraus sein kann, wobei A ausgewählt aus Cs, CH₃NH₃, CH(NH₂)₂, Ca, Sr, Bi, La, Ba, Mg und/oder Mischungen daraus sein kann, wobei B ausgewählt aus Pb, Sn, Sr, Ge, Mg, Ca, Bi, Ti, Mn, Fe und/oder Mischungen daraus sein kann.

Des Weiteren sind Kern/Schale und/oder Kern/Multischalen aus halbleitenden anorganischen Nanokristall-Architekturen aus II-VI, III-V, IV-VI, I-VI, I-III-VI Halbleitern oder Mischungen daraus sowie Kern/Schale und/oder Kern/Multischalen aus Perovskite-Materialien, weitere geeignete Beispiele.

Das Kristallgitter der halbleitenden anorganischen Nanokristalle kann zusätzlich, aber nicht ausschließlich, mit einem oder mehreren Metallionen, wie beispielsweise Cu⁺, Mg²⁺, Co²⁺, Ni²⁺, Fe²⁺, Mn²⁺ und/oder mit einem oder mehreren Seltenerdmetallen, wie beispielsweise Ytterbium, Praeseodym oder Neodym, dotiert sein.

Die halbleitenden anorganischen Nanokristalle haben bevorzugt eine durchschnittliche Partikelgröße von 1 nm bis 100 nm, stärker bevorzugt von 2 nm bis 50 nm und am stärksten bevorzugt von 3 nm bis 15 nm in mindestens eine Dimension, vorzugsweise in alle Dimensionen.

Die durchschnittliche Partikelgröße kann durch verschiedene Methoden noch vergrößert/modifiziert werden. Typische Beispiele sind, aber nicht ausschließlich, eine Silica-Schale, eine Titanoxid-Schale, eine Halogen-Schale sowie weiteren Verfahren zur Stabilitätserhöhung, Maskierung, Bioverträglichkeit, Wasserlöslichkeit und/oder Umhüllung.

Eine für die vorliegende Erfindung interessante Eigenschaft der halbleitenden anorganischen Nanokristalle ist, dass deren Anregungs- und Emissionsspektrum unter anderem abhängig von deren Partikelgröße ist.

Des Weiteren können auch "Anti-Stoke-shift"-Materialien im Sinne der Erfindungen eingesetzt werden. Diese Leuchtstoffe sind gewöhnlich mit den Elementen Scandium und Yttrium sowie den Elementen der Lanthanide- oder Actinide-Gruppe gedopt. Die chemische Zusammensetzung dieser Leuchtstoffe setzt sich aus dem Wirtsgitter, den Donor- und den Akzeptor-Ionen zusammen. Die chemische Zusammensetzung beeinflusst ihre spektralen Eigenschaften.

Im Sinne der Erfindung können auch phosphoreszierende Materialien eingesetzt werden. Phosphoreszierende Materialien sind meist Kristalle, welche mit einem Dopenden verunreinigt sind.

Phosphoreszenzfarbstoffe sind üblicherweise ausgewählt aus dotierten Oxiden, Nitriden, Oxynitriden, Sulfiden, Seleniden, Haliden, Silikaten und Aluminaten von Calcium, Strontium, Barium, Zink, Cadmium, Mangan, Silizium und Seltenerdmetallen und Mischungen daraus. Meistens, aber nicht ausschließlich verwendet man Sulfide von Metallen der zweiten Hauptgruppe des Periodensystems und Zink sowie Aluminate von Metallen der zweiten Hauptgruppe des Periodensystems. Die Dopenden können z.B. Metalle oder Metallsalze darstellen. Geeignete Beispiele für Phosphoreszenzfarbstoffe sind dotierte Sulfiden und Aluminate von Calcium, Strontium, Barium und Zink, wie beispielsweise mit Bismut dotiertes Calcium/Strontiumsulfid, mit Kupfer dotiertes Zinksulfid, sowie mit Europium dotiertes Strontiumaluminat.

Die in der erfindungsgemäßen Methode verwendeten Photolumineszenzfarbstoffe mit einem "Stoke-Stift"-Verhalten sind vorzugsweise photolumineszierende Substanzen, die durch Lichtabsorption eines höher energetischen Photons in elektronisch angeregte Energiezustände gebracht werden, und daraufhin unter Aussenden von Licht in Form von Fluoreszenz oder Phosphoreszenz wieder energetisch tiefer liegende Energiezustände erreichen.

Die in der erfindungsgemäßen Methode verwendeten Photolumineszenzfarbstoffe mit einem "Anti-Stoke-shift"-Verhalten sind vorzugsweise photolumineszierende Substanzen, die durch Lichtabsorption von zwei niedrig- energetischen Photonen ein Lichtquant mit höherer Energie aussenden. Üblicherweise gibt es zwei Prozesse, um ein "Anti-Stoke-shift"-Verhalten zu generieren. Auf der einen Seite werden die Pigmente mittels hoher Photonenfluss (üblicherweise mit Laser) bestrahlt, sodass ein zwei niedrig- energetischen Photonen ein Lichtquant mit höherer Energie aussenden können.

Auf der anderen Seite werden die Pigmente mittels Photonenfluss bestrahlt, sodass ein metastabilen Überstandzustand generiert wird. Durch nochmalige Absorption eines Photons kann nun ein Lichtquant höherer Energie aussenden werden.

Die Photolumineszenzfarbstoffe mit "Stoke-shift"-Verhalten werden bevorzugt von UV-Licht, sichtbarem Licht, wie blauem oder weißem Licht, sowie höher energetischer nahinfrarot-Strahlung als das Emissionssignal angeregt.

Die Photolumineszenzfarbstoffe mit "Anti-Stoke-shift"-Verhalten werden bevorzugt von nahinfrarot- (NIR-)Strahlung, insbesondere Strahlung der Wellenlängen zwischen 750 - 1600 nm angeregt.

Die Photolumineszenzfarbstoffe emittieren unter Photonenanregung eine Strahlung mit einer Wellenlänge im Bereich von 380 bis 3000 nm, stärker bevorzugt von 450 bis 1800 nm, am stärksten bevorzugt von 750 nm bis 1100 nm.

Der Anteil der Photolumineszenzfarbstoffe in der Tintenformulierung beträgt unabhängig voneinander jeweils 0,01 bis 70,0 Gewichts-%, stärker bevorzugt 0,05 bis 40,0 Gewichts-%, am stärksten bevorzugt 0,09 bis 30,0 Gewichts-%, gemessen am Gesamtgewicht der Tintenformulierung. Für Digital- und Inkjetdruck ist ein Bereich zwischen 0,01 - 30,0 Gewichts-% zu bevorzugen.

Die Tintenformulierungen können Photolumineszenzfarbstoffe enthalten, die mindestens einen oder mehrere, vorzugsweise alle, der folgenden Eigenschaften gemeinsam haben: Emissionswellenlänge, Emissionsverteilung, Emissionsmaximum. In einer anderen Ausführungsform können die Tintenformulierungen Mischungen von Photolumineszenzfarbstoffen enthalten, die unterschiedliche Werte haben bei Emissionswellenlänge, Emissionsverteilung und Emissionsmaximum.

Weiterhin können die Tintenformulierungen die Farbpigmente der kommerziellen Tinten enthalten. Es können kommerzielle Tintenformulierungen genutzt werden und diese mit den Photolumineszenzfarbstoffen versetzt werden.

Die emittierte Strahlung der Tintenformulierungen können ein individuelles Photolumineszenzspektrum ergeben, das abhängig ist von der Art, Menge, Partikelgröße, Eigenfarbe und besonders der Photolumineszenz-Lebensdauer der Photolumineszenzfarbstoffe.

Dabei kann das individuelle Photolumineszenzspektrum mit einem Spektrometer detektiert werden. Das detektierten individuelle Photolumineszenzspektrum kann dann mit einem in einer Datenbank hinterlegten Referenzspektrum verglichen werden. Das individuelle Photolumineszenzspektrum enthält durch die Verwendung von Photolumineszenzfarbstoffen mit unterschiedlichen Photolumineszenzlebensdauern eine zeitliche Komponente, die dazu führt, dass sich das individuelle Photolumineszenzspektrum über die Zeit, vorzugweise einen Zeitraum von 1 ns bis 1 min, bevorzugt 1 ns bis 1 s, stärker bevorzugt 1 ns bis 1 ms, am stärksten bevorzugt 5 ns bis 100 µs nach Beginn der Bestrahlung verändert. Diese Veränderungen lassen sich mit verschiedenen Methoden abbilden und vergleichen. Zum einen können über einen Zeitraum von 1 ns bis 1 min, bevorzugt 1 ns bis 1 s, stärker bevorzugt 1 ns bis 1 ms, am stärksten bevorzugt 5 ns bis 100 µs nach Beginn der Bestrahlung zu festgelegten Zeitpunkten Messungen des jeweiligen Photolumineszenzspektrums gemacht werden, die dann mit entsprechenden in einer Datenbank hinterlegten Referenzspektren von den gleichen Zeitpunkten verglichen werden. Alternativ kann über einen Zeitraum von 10 ns bis 1 s, bevorzugt 20 ns bis 100 ms, stärker bevorzugt 50 ns bis 10 ms, am stärksten bevorzugt 75 ns bis 1 ms nach Beginn der Bestrahlung ein "Film" des individuellen Fluoreszenzspektrums aufgenommen werden und mit einem in einer Datenbank hinterlegten Referenzfilm verglichen werden.

Dieses individuelle zeitabhängige Fluoreszenzspektrum kann als weiteres Sicherheitsmerkmal für eine vom Produzenten des Produkts individuell zusammengestellte Anzahl verschiedener Tintenformulierungen eingesetzt werden.

Die Tintenformulierungen haben jeweils vorzugsweise eine reziproke Ohnesorgzahl von weniger als 14, stärker bevorzugt von 1 bis 10, noch stärker bevorzugt von 1 bis 8 und am stärksten bevorzugt von 2 bis 4.

In einem weiteren Schritt wird ein mehrdimensionaler Code zur Identifizierung eines Produkts generiert, wobei mindestens eine Dimension, bevorzugt zwei Dimensionen, eine örtliche Dimension ist/sind und eine Dimension eine zeitliche Dimension ist, beruhend auf den Photolumineszenz-Lebensdauern der Photolumineszenzfarbstoffe.

Geeignete Dimensionen für den mehrdimensionalen Code sind örtliche Dimensionen, beispielsweise in x- und/oder y-Richtung, farbliche Dimensionen, beispielsweise die Eigenfarbe der Farbstoffe auf einem Substrat als mehrfarbiger Code und/oder die Photolumineszenz der Photolumineszenzfarbstoffe, welche in den Raum und auf den Substrat abgestrahlt wird, sowie die zeitliche Dimension über die Photolumineszenz-Lebensdauer der Photolumineszenzfarbstoffe.

Der mehrdimensionale Code kann ein dreidimensionaler, vierdimensionaler, fünfdimensionaler sein.
Beispiel eines dreidimensionalen Codes ist
   - ein Strichcode, der Photolumiszenzfarbstoffe enthält, wobei die Photolumineszenzfarbstoffe auch verschiedene Photolumineszenz-Lebensdauern aufweisen (1 örtliche Dimension, 1 farbliche Dimension, 1 zeitliche Dimension).
Beispiele eines vierdimensionalen Codes sind:
   - ein mehrfarbiger Strichcode, der Photolumiszenzfarbstoffe enthält, wobei die Photolumineszenzfarbstoffe auch verschiedene Photolumineszenz-Lebensdauern aufweisen (1 örtliche Dimension, 2 farbliche Dimensionen, 1 zeitliche Dimension);
   - ein QR-Code, der Photolumiszenzfarbstoffe enthält, wobei die Photolumiszenzfarbstoffe auch verschiedene Photolumineszenz-Lebensdauern aufweisen (2 örtliche Dimensionen, 1 farbliche Dimension, 1 zeitliche Dimension);
Beispiel eines fünfdimensionalen Codes ist:
   - ein mehrfarbiger QR-Code, der Photolumiszenzfarbstoffe enthält, wobei die Photolumiszenzfarbstoffe auch verschiedene Photolumineszenz-Lebensdauern aufweisen (2 örtliche Dimensionen, 2 farbliche Dimension, 1 zeitliche Dimension).

Der mehrdimensionale Code kann auch ein oder mehrere Muster, wie beispielsweise Flächen, Streifen, Linien, geometrische Figuren, wie Kreise, Dreiecke, Rechtecke, Vielecke etc, alphanummerische Zeichen, oder Kombinationen daraus, enthalten.

Der mehrdimensionale Code kann auch ein einzigartiger mehrdimensionaler Code sein.

Hierzu kann vorzugsweise zunächst mindestens eine Bezugsgröße, bevorzugt mehrere Bezugsgrößen des Produkts mithilfe eines einzigartigen Schlüssels verschlüsselt werden.

Mögliche Bezugsgrößen sind dabei beispielsweise Bezugsgrößen zu Art und Beschaffenheit des Produkts wie Seriennummern, Lot-Nummern, CAS-Nummer bei chemischen Produkten, zum Produktionsort, zum Produktionszeitpunkt, zum Lieferort, zum Produzenten, zum Lieferanten, zum Abnehmer oder ähnliche.

Der einzigartige Schlüssel kann ein dem Produzenten zur Verfügung gestellter oder vom Produzenten selbst erstellter Algorithmus sein.

Über die Verschlüsselung wird ein für das Produkt, vorzugsweise für die individuelle Packungseinheit des Produkts, einzigartiger Code generiert.

Die Tintenformulierungen werden auf mindestens eine Fläche der Oberfläche des Produktes in Form des mehrdimensionalen Codes gedruckt.

Bei Generation eines einzigartigen mehrdimensionalen Codes wird vorzugsweise jede Packungseinheit des Produkts mit einem eigenen einzigartigen mehrdimensionalen Code bedruckt.

In einer speziellen Ausführungsform wird ein zusätzlicher einzigartiger mehrdimensionaler Code beim Druckprozess durch ein individuelles Druckmuster - bedingt durch Fehlstellen im individuellen Druckmuster, z.B. durch die hohe Druckfrequenz und Ablenkungen der Tintentropfen - generiert. Hierbei wird durch jeden Druckprozess ein tatsächlich einzigartiger Code generiert, da sich die Fehlstellen im individuellen Druckmuster nicht reproduzieren lassen.

Der Schritt "Drucken der Tintenformulierungen auf mindestens eine Fläche der Oberfläche des Produktes in Form dieses mehrdimensionalen Codes" umfasst dabei sowohl den Druck der Tintenformulierungen direkt auf mindestens eine Fläche der Oberfläche des Produktes, soweit die Gegenständlichkeit des Produktes dies zulässt, als auch das Drucken der Tintenformulierungen auf mindestens ein Etikett in Form dieses mehrdimensionalen Codes und Bekleben/Etikettieren der Oberfläche des Produkts mit mindestens einem bedruckten Etikett.

Falls die Form und/oder Gegenständlichkeit des Produktes eine direkte Kennzeichnung nicht zulässt, kann der Schritt "Drucken der Tintenformulierungen auf mindestens eine Fläche der Oberfläche des Produktes in Form dieses mehrdimensionalen Codes" auch den Druck der Tintenformulierungen direkt auf mindestens eine Fläche der Oberfläche der Verpackung des Produktes oder Bekleben/Etikettieren der Oberfläche des Produkts mit mindestens einem bedruckten Etikett umfassen.

Der mehrdimensionale Code kann auch auf Dokumente gedruckt werden.

Hierzu sind die üblichen Druckmethoden abhängig von der Art der Tintenformulierungen anwendbar. Vorzugsweise werden die Tintenformulierungen mittels Digitaldruck, Siebdruck, Transferdruck, Rolle zu Rolle Druckverfahren, "Druck ohne Kontakt"-Verfahren oder Laserdruck auf mindestens eine Fläche der Oberfläche des Produktes oder Dokuments gedruckt.

Abhängig von der Art des Produkts kann der mehrdimensionale Code direkt auf die Oberfläche des Produkts oder Dokuments, auf die Verpackung des Produkts sowie auf Etiketten, Schilder, Barcode-Karten und/oder Barcode-Etiketten gedruckt werden.

Zusätzlich zu dem mehrdimensionalen Code können die Tintenformulierungen auch in anderen Mustern und einzigartigen Codes, wie beispielsweise Flächen, Streifen, Linien, geometrische Figuren, wie Kreise, Dreiecke, Rechtecke, Vielecke etc., alphanummerischen Zeichen, oder Kombinationen daraus, auf mindestens einer Fläche der Oberfläche des Produktes gedruckt werden. Das aufgedruckte Muster kann hierbei als reines zeitabhängiges Authentifizierungsmerkmal dienen oder Informationen, wie Sicherheits- und Gebrauchshinweise oder Herstellerinformationen, enthalten.

Die Tintenformulierungen werden vorzugsweise nebeneinander gedruckt und bilden so eine Schicht aus Punkten unterschiedlicher Photolumineszenzfarbstoffzusammensetzung, die ein zeitabhängiges Muster des mehrdimensionalen Codes ergeben.

In manchen Ausführungsformen werden die Tintenformulierungen nebeneinander und optional übereinander gedruckt und bilden so eine und/oder mehrere Schichten aus Punkten unterschiedlicher Photolumineszenzfarbstoffzusammensetzung, die ein zeitabhängiges Muster des mehrdimensionalen Codes ergeben.

In einem weiteren Schritt wird das mit den Tintenformulierungen bedruckte Produkt mit Photonen bestrahlt.

Durch die Photonenbestrahlung werden die in den Tintenformulierungen befindlichen Photolumineszenzfarbstoffe in angeregte Energiezustände gebracht (Anregung).

Vorzugsweise wird das mit den Tintenformulierungen bedruckte Produkt mit ultraviolettem, sichtbarem Licht, wie blauem oder weißem Licht, oder NIR-Licht bestrahlt, bevorzugt mit ultraviolettem, blauem oder weißen Licht.

Als Lichtquelle dient beispielsweise eine Schwarzlichtlampe, Halogenlampe oder LED Lampe, bevorzugt eine blaue oder weiße LED Lampe. Eine geeignete Lichtquelle für die Bestrahlung ist zudem ein LED-Blitz, wie beispielsweise der LED-Blitz eines Endgerätes, wie z.B. eines Smartphones oder Tablets.

Nach Bestrahlung emittiert das bestrahlte Produkt, vorzugsweise Photolumineszenzfarbstoffe in den Tintenformulierungen, eine Strahlung im Bereich von 380 bis 3000 nm, bevorzugt von 450 bis 1800 nm, am stärksten bevorzugt von 750 nm bis 1100 nm. Diese wird in einem weiteren Schritt über einen Zeitraum von 1 ns bis 1 min, bevorzugt 1 ns bis 1 s, stärker bevorzugt 1 ns bis 1 ms, am stärksten bevorzugt 5 ns bis 100 µs nach Beginn der Bestrahlung detektiert.

Üblicherweise ist die Bestrahlungsdauer kürzer als die Photolumineszenzlebensdauer der Photolumineszenzfarbstoffe.

Die Bestrahlung kann dabei entweder über gepulste Photonenbestrahlung oder über kontinuierliche Photonenbestrahlung erfolgen.

Gepulste Photonenbestrahlung erfolgt üblicherweise bei der Verwendung von Photolumineszenzfarbstoffen mit kurzen Photolumineszenz-Lebensdauern bis zu einigen ms, bevorzugt bei der Verwendung von Fluoreszenzfarbstoffen. Kontinuierliche Photonenbestrahlung erfolgt üblicherweise bei der Verwendung von Photolumineszenzfarbstoffen mit längeren Photolumineszenz-Lebensdauern ab einigen ms, bevorzugt bei der Verwendung von Phosphoreszenzfarbstoffen.

Die emittierte Strahlung kann mit jedem dazu geeigneten Detektionsgerät detektiert werden.

In der zeitlichen Dimension wird das individuelle Photolumineszenzspektrum des mehrdimensionalen Codes über einen Zeitraum von 1 ns bis 1 min, bevorzugt 1 ns bis 1 s, stärker bevorzugt 1 ns bis 1 ms, am stärksten bevorzugt 5 ns bis 100 µs nach Beginn der Bestrahlung beobachtet und Veränderungen über diesen Zeitraum notiert. Diese Veränderungen beruhen auf den unterschiedlichen Photolumineszenz-Lebensdauern der verwendeten Photolumineszenzfarbstoffen, die dafür sorgen, dass emittierte Strahlung eines Photolumineszenzfarbstoffs früher abklingt als die eines anderen und es somit über die Zeit zu einer Veränderung des Musters des mehrdimensionalen Codes kommt.

Diese Veränderungen können punktuell notiert und abgeglichen werden, beispielsweise indem während der Detektionsdauer zu festgelegten Zeitpunkten zwei oder mehrere Spektren der emittierten Strahlung aufgenommen werden.

Es können zusätzlich zu oder anstelle von Spektren auch Aufnahmen der emittierten Strahlung zu den jeweiligen festgelegten Zeitpunkten gemacht werden und dann die Veränderungen in diesen Aufnahmen abgeglichen werden.

Alternativ können diese Veränderungen kontinuierlich notiert werden, beispielsweise indem während der Detektionsdauer ein Film der emittierten Strahlung gemacht wird.

Dabei können verschiedene Parameter des Musters des mehrdimensionalen Codes sowie deren Veränderung notiert werden, wie beispielsweise Veränderungen des Musters in x- und y-Richtung, Farbe, Photolumineszenz oder deren Lebensdauer. Diese Parameter können dann in mindestens einer Datenbank gespeichert werden und aus dieser abgefragt werden. Die gemessenen Parameter können dann mit den in der Datenbank gespeicherten Parametern abgeglichen werden.

Das Muster des mehrdimensionalen Codes kann somit als optisches zeitabhängiges Authentifizierungsmerkmal verwendet werden.

Das erfindungsgemäße Verfahren kann somit folgende weitere Schritte enthalten:
- Speichern des mehrdimensionalen Codes in mindestens einer Datenbank;
- Abfragen des mehrdimensionalen Codes aus der Datenbank und
- Abgleich dieses Codes mit dem detektierten Code zur Verifizierung oder Authentifizierung des Produkts.

Die Methode lässt sich weiterhin auch in Serialisierungs- und/oder Track & Tracesystemen verwenden.

Bei der Serialisierung werden strukturierte Daten auf eine sequentielle Darstellungsform abgebildet. Serialisierung wird hauptsächlich für die Übertragung von Objekten über das Netzwerk bei verteilten Softwaresystemen verwendet.

Zur Verwendung in Serialisierungssystemen sind folgende weitere Schritte bevorzugt:
- Speichern des einzigartigen mehrdimensionalen Codes in mindestens einer Datenbank;
- Abfragen des einzigartigen mehrdimensionalen Codes aus der Datenbank und
- Abgleich dieses Codes mit dem detektierten Code zur Verifizierung des Produkts.

In weiterführenden Serialisierungssystemen können ein oder mehrere Bezugsgrößen eines Produkts erfasst und/oder mit Hilfe eines einzigartigen Schlüssels verschlüsselt werden. Über ein entsprechendes Serialisierungs- und/oder Track & Trace-Computerprogramm wird ein einzigartiger mehrdimensionaler Code generiert, der auf das Produkt aufgedruckt wird. Zusätzlich wird der Code in einer Datenbank, vorzugsweise einer zentralen Datenbank gespeichert. Der Code kann dann jederzeit gescannt werden und aus der Datenbank ausgelesen werden. Über das Serialisierungs- und/oder Track & Trace-Computerprogramm können somit die verschlüsselten Bezugsgrößen des Produkts ausgelesen werden.

Zur Verwendung in Track & Tracesystemen ist weiterhin bevorzugt, dass die Tintenformulierung zusätzlich auf mindestens eine Fläche der Oberfläche einer Verpackungsgruppe, die das Produkt enthält, beispielsweise ausgewählt aus Bündeln, Umverpackung, Paletten, in Form des einzigartigen Codes aufgedruckt wird.

Dies ermöglicht eine lückenlose Verfolgung des Produkts während des Produktions- und Transportweges des einzelnen Produkts.

Die vorliegende Methode stellt somit eine Kombination aus Track & Trace Technologie und optischen Sicherheitsmerkmalen dar. So werden der Rückverfolgungsprozess und der Authentifizierungsprozess von Produkten miteinander vereinigt.

Weiterhin kann die Methode zur Sicherung von Dokumenten, wie Banknoten, Ausweise, Pässe, Geburtsurkunden, Fahrausweise, Eintrittskarten und andere Tickets, oder andere Dokumente wie hierin beschrieben verwendet werden.

Figur 1 zeigt einen Überblick über eine mögliche Ausführungsform der erfindungsgemäßen Methode in einem Track & Trace-System.

Hierbei werden in einem ersten Schritt Bezugsgrößen eines Produkts, wie beispielsweise Produktionsort und -zeitraum, Inhaltsstoffe des Produkts, Darreichungsformen etc., identifiziert.

Anschließend werden diese Bezugsgrößen in einer Eigenschaftsmatrix mit Hilfe der unterschiedlichen Fluoreszenzlebensdauern der verschiedenen Fluoreszenzfarbstoffe verknüpft und so ein für das Produkt einzigartiger mehrdimensionaler Code erstellt. Dieser Code kann ein zweidimensionaler, dreidimensionaler oder vierdimensionaler Code sein, z.B. ein Strichcode, ein QR Code oder ein farbiger Barcode, jeweils mit einer an die unterschiedlichen Fluoreszenzlebensdauern der verschiedenen Fluoreszenzfarbstoffe geknüpften zeitlichen Dimension. Der Code wird auf die Oberfläche des Produkts gedruckt mithilfe der hierin offenbarten Tintenformulierungen mit den für die Eigenschaftsmatrix verwendeten Fluoreszenzfarbstoffen. Abhängig von dem Produkt kann der Code direkt auf die Oberfläche des Produkts oder auf die Verpackung des Produkts gedruckt werden. Informationen aus der Eigenschaftsmatrix werden, beispielsweise über ein Track & Trace Computerprogramm, in einer zentralen Datenbank gespeichert.

Das Produkt kann nun weitergegeben werden, beispielsweise in eine neue Produktionsstufe oder in den Verkauf.

In der neuen Umgebung wird das Produkt nun zur Identifizierung gescannt und der Code ausgelesen.

Der mithilfe der hierin offenbarten Tintenformulierungen gedruckte Code kann nun als Track & Trace-Kennzeichnung verwendet werden.

Der Code wird in das Track & Trace Computerprogramm übermittelt. Der Code wird dabei aus der Datenbank ausgelesen. Somit werden die Bezugsgrößen des gekennzeichneten Produkts erhalten.

Der Code sowie alle weiteren möglichen Kennzeichnungen mit den hierin offenbarten Tintenformulierungen kann/können auch als optische Authenifizierungskennzeichnung verwendet werden (wie gezeigt in Figur 1). Hierzu wird die Oberfläche des Produkts mit Licht, vorzugsweise weißem oder blauem Licht. Die photolumineszierende Substanz, vorzugsweise die Fluoreszenzfarbstoffe in den Tintenformulierungen, werden hierbei, wie oben diskutiert, angeregt und emittieren dann Photolumineszenzstrahlung im Bereich von 400-1800 nm (sichtbare und NIR-Strahlung). Diese Strahlung kann nur teilweise (bis ca. 800 nm) mit dem menschlichen Auge nicht wahrgenommen werden. Zur Detektion ist stattdessen ein elektronisches Gerät notwendig, dass die sichtbare und NIR-Photolumineszenzstrahlung detektieren kann. Geeignet wären beispielsweise Spektrometer oder NIR-Kameras.

Die Anregung und die Detektion kann so gesteuert werden, so dass nach Anregung und Detektion mehrere zu festgelegten Zeitpunkten über einen Zeitraum von 1 ns bis 1 min, bevorzugt 1 ns bis 1 s, stärker bevorzugt 1 ns bis 1 ms, am stärksten bevorzugt 5 ns bis 100 µs nach Beginn der Bestrahlung gemachten Messungen oder ein über diesen Zeitraum aufgenommener Film des Codes auf dem Bildschirm des Endgeräts erscheint. Dieses Foto dient somit als optisches zeitabhängiges Authentifizierungsmerkmal und erlaubt die zeitabhängige Authentifizierung des Produkts.

Bei Anwendung in einem Serialisierungs- oder Track & Trace-System erweitert die erfindungsgemäße Methode somit dieses System um ein zeitabhängiges optisches Sicherheitsmerkmal, dass für das menschliche Auge teilweise nicht sichtbar ist (750-3000 nm).

Die erfindungsgemäße Methode erhöht durch die zeitliche Dimension die Sicherheit, weil ein individuelles zeitabhängiges Photolumineszenzspektrum im sichtbaren und NIR Bereich emittiert wird, das mithilfe eines Spektrometers detektiert werden kann. Zusätzlich erhöht die erfindungsgemäße Methode die Komplexität und das Speichervolumen des Codes durch die zusätzlich nutzbaren Dimensionen des zeitabhängigen Musters. Dieses individuelle zeitabhängige Photolumineszenzspektrum kann wiederum als zusätzliches Authentifizierungsmerkmal verwendet werden. Durch dieses zeitabhängige Authentifizierungsmerkmal kann das Sicherheitsmerkmal, z.B. ein QR-Code mit zeitabhängigem zweitem Faktor, als CPS der Stufe Sichere Identität in der Industrie 4.0 und Logistik 4.0 (Lieferkettensicherheit) verwendet werden.

Gegenüber anderen Authentifizierungsmerkmalen wie RFID-Chips oder Hologrammen hat die erfindungsgemäße Methode auch einen klaren Kostenvorteil.

Die vorliegende Erfindung betrifft zudem auch ein optisches, zeitabhängiges Sicherheitsmerkmal auf mindestens einer Fläche der Oberfläche eines Produktes in Form eines mehrdimensionalen Codes, das zwei oder mehrere Fluoreszenzfarbstoffe enthält, die unter Photonenanregung eine Strahlung im Bereich von 380-3000 nm emittieren und sich durch verschiedene Fluoreszenzlebensdauern unterscheiden. Weiterhin betrifft die Erfindung ein optisches, zeitabhängiges Sicherheitsmerkmal auf mindestens einer Fläche der Oberfläche eines Produktes in Form eines mehrdimensionalen Codes, das Photolumineszenzfarbstoffe enthält, die unter Photonenanregung eine Strahlung im Bereich von 380-3000 nm emittieren und sich in ihren Photolumineszenzlebensdauern unterscheiden.

Das optische, zeitabhängige Sicherheitsmerkmal wird dabei mit der erfindungsgemäßen Methode auf mindestens einer Fläche der Oberfläche des Produktes aufgedruckt.

Weiterhin betrifft die Erfindung die Verwendung des optischen, zeitabhängigen Sicherheitsmerkmal wie hierin beschrieben als cyber-physical system (CPS) zur Produktüberwachung.

Zudem betrifft die Erfindung die Verwendung des optischen, zeitabhängigen Sicherheitsmerkmal wie hierin beschrieben als cyber-physical system (CPS) zur Dokumentensicherung.

Die vorliegende Erfindung betrifft weiterhin ein Serialisierungs- und/oder Track & Tracesystem, das ein optisches, zeitabhängiges Sicherheitsmerkmal beinhaltend einen auf ein Produkt aufgedruckten mehrdimensionalen Code wie hierin beschrieben enthält.

Zusätzlich betrifft die Erfindung die Verwendung eines auf ein Produkt aufgedruckten mehrdimensionalen Codes wie hierin beschrieben als optisches, zeitabhängiges Sicherheitsmerkmal in einem Serialisierungs- und/oder Track & Tracesystem.

Zusätzlich betrifft die Erfindung die Verwendung eines auf ein Produkt aufgedruckten mehrdimensionalen Codes wie hierin beschrieben als optisches, zeitabhängiges Sicherheitsmerkmal zur Dokumentensicherung.

Dabei erhöht die erfindungsgemäße Methode durch die zeitliche Dimension die Sicherheit, weil ein individuelles zeitabhängiges Photolumineszenzspektrum im sichtbaren und NIR Bereich emittiert wird, das mithilfe eines Spektrometers detektiert werden kann. Zusätzlich erhöht die erfindungsgemäße Methode die Komplexität und das Speichervolumen des Codes durch die zusätzlich nutzbaren Dimensionen des zeitabhängigen Musters.

Der einzigartige mehrdimensionale Code wird hierbei mithilfe der hierin beschriebenen Tintenformulierungen, die Photolumineszenzfarbstoffe enthält, die unter Photonenanregung eine Strahlung im Bereich von 380-3000 nm emittieren und sich durch verschiedene Photoluminezenzlebensdauern unterscheiden, auf das Produkt oder die Produktverpackung oder ein Dokument aufgedruckt.

Die hierin beschriebenen Merkmale des Codes, der Tintenformulierungen und der Photolumineszenzfarbstoffe sind auch auf das erfindungsgemäße optische, zeitabhängige Sicherheitsmerkmal, das erfindungsgemäße Serialisierungs- und/oder Track & Tracesystem sowie die erfindungsgemäßen Verwendungen anzuwenden. Ebenso sind die hierin beschriebenen Merkmale des Serialisierungs- und/oder Track & Tracesystem anzuwenden.

Figur 2 zeigt ein Beispiel eines gedruckten vierdimensionalen Codes. Der Code besteht aus einem Quadrat aus 5 x 5 nebeneinander gedruckten Quadraten aus verschiedenen Tintenformulierungen. Die fünf verschiedenen Graustufen der gedruckten Quadrate deuten auf Tintenformulierungen mit fünf verschiedenen Fluorenzfarben hin. In hervorgehobenen unterschiedlichen Quadraten mit einer Fluoreszenzfarbe, angedeutet in dunklem Grau, wurden zudem Tintenformulierungen mit verschiedenen Fluoreszenzfarbstoffen verwendet, die unterschiedliche Fluoreszenzlebensdauern besitzen. Diese sind in den Abklingdiagrammen dargestellt als Counts über die Zeit in ns.

Im linken Abklingspektrum wurden halbleitenden Nanopartikel aus CdSeCdS mit einem Emissionsmaximum bei 621 nm und eine Fluoreszenzlebensdauer von 40 ns eingesetzt.

Im mittleren Abklingspektrum wurden halbleitenden Nanopartikel aus CdSeCdS mit einem Emissionsmaximum bei 623 nm und eine Fluoreszenzlebensdauer von 89 ns eingesetzt.

Im rechten Abklingspektrum wurden halbleitenden Nanopartikel aus CdSeCdS mit einem Emissionsmaximum bei 625 nm und eine Fluoreszenzlebensdauer von 157 ns eingesetzt.

Der Code umfasst somit zwei räumliche Dimensionen in x- und y-Richtung, eine farbliche Dimension über fünf verschiedene Fluoreszenzfarben der Fluoreszenzfarbstoffe, und eine zeitliche Dimension über die verschiedenen Fluoreszenzlebensdauern der Fluoreszenzfarbstoffe.

Figur 3 zeigt ein Beispiel einer zeitlichen Veränderung eines vierdimensionalen Codes. Auch hier besteht der Code aus einem Quadrat aus 5 x 5 nebeneinander gedruckten Quadraten aus verschiedenen Tintenformulierungen. Die fünf verschiedenen Farben der gedruckten Quadrate deuten auf Tintenformulierungen mit fünf verschiedenen Fluoreszenzfarben. Die zeitliche Dimension zeigt sich aus Aufnahmen zu verschiedenen Zeitpunkten nach Beginn der Bestrahlung.

Direkt nach Beginn der Bestrahlung (0 ns) sind alle 25 gedruckten Quadrate farbig zu erkennen (links).

25 ns nach Beginn der Bestrahlung zeigt sich bei verschiedenen Quadraten ein unterschiedliches Abklingverhalten. Während einige Quadrate eine nahezu unveränderte Fluoreszenz abstrahlen, weisen andere Quadrate eine wahrzunehmende Abschwächung auf und die Fluoreszenz von fünf Quadraten ist bereits im messbaren Bereich erloschen, was durch ein schwarzes Quadrat symbolisiert ist (Mitte). Das unterschiedliche Abklingverhalten der Fluoreszenzstrahlung weist auf unterschiedliche Fluoreszenzlebensdauern der Fluoreszenzfarbstoffe hin, wobei die schwarzen Quadrate auf sehr kurze Fluoreszenzlebensdauern hinweisen, die abgeschwächten Quadrate auf mittlere Fluoreszenzlebensdauern und die unveränderten Quadrate auf längere Fluoreszenzlebensdauern der Fluoreszenzfarbstoffe.

100 ns nach Beginn der Bestrahlung sind emittieren nur noch die fünf Quadrate aus Fluoreszenzfarbstoffen mit der längsten Fluoreszenzlebensdauer Fluoreszenz. Diese stilisieren einen Smiley. So kann über die zeitliche Dimension des Codes ein weiteres einfaches Authentifizierungsmerkmal eingeführt werden, indem die Fluoreszenzfarbstoffe mit den verschiedenen Fluoreszenzlebensdauern so im Code arrangiert werden, dass je nach Abklingzeit unterschiedliche Bilder und Symbole sichtbar werden, die als Authentifizierungsmerkmal verwendet werden können.

## Patentansprüche

1. Methode zur Kennzeichnung von Produkten, die folgende Schritte enthält:
- Bereitstellung von Tintenformulierungen, die ein oder mehrere Photolumineszenzfarbstoffe, bevorzugt einen Photolumineszenzfarbstoff, enthalten, die unter Photonenanregung eine Strahlung im Bereich von 380-3000 nm emittieren, bevorzugt von 450 bis 1800 nm, am stärksten bevorzugt von 750 nm bis 1100 nm,;
- Generierung eines mehrdimensionalen Codes zur Identifizierung eines Produkts, wobei mindestens eine Dimension, bevorzugt zwei Dimensionen, eine örtliche Dimension ist/sind ;
- Drucken der Tintenformulierungen auf mindestens eine Fläche der Oberfläche des Produktes in Form dieses mehrdimensionalen Codes;
- Bestrahlung des mit der Tintenformulierung bedruckten Produktes mit Photonen;
- Detektion der von dem bestrahlten Produkt emittierten Strahlung im Bereich von 380 bis 3000 nm, bevorzugt von 450 bis 1800 nm, am stärksten bevorzugt von 750 nm bis 1100 nm,
wobei zwei oder mehr Tintenformulierungen bereitgestellt werden, die alle jeweils ein oder mehrere Photolumineszenzfarbstoffe, bevorzugt einen Photolumineszenzfarbstoff, enthalten, wobei sich die Tintenformulierungen durch verschiedene Photolumineszenz-Lebensdauern der Photolumineszenzfarbstoffe unterscheiden;
eine Dimension des mehrdimensionalen Codes eine zeitliche Dimension ist, beruhend auf den Photolumineszenz-Lebensdauern der Photolumineszenzfarbstoffe;
**dadurch gekennzeichnet, dass**
der zeitliche Verlauf der von dem bestrahlten Produkt emittierten Strahlung über einen Zeitraum von 1 ns bis 1 min, bevorzugt 1 ns bis 1 s, stärker bevorzugt 1 ns bis 1 ms, am stärksten bevorzugt 5 ns bis 100 µs nach Beginn der Bestrahlung detektiert wird,
und dass der Anteil der Photolumineszenzfarbstoffe in den Tintenformulierungen unabhängig voneinander jeweils 0,01 bis 70,0 Gewichts-%, bevorzugt 0,05 bis 40,0 Gewichts-%, am stärksten bevorzugt 0,09 bis 30,0 Gewichts-%, gemessen am Gesamtgewicht der jeweiligen Tintenformulierung beträgt.

2. Die Methode gemäß Anspruch 1, wobei der mehrdimensionale Code ein dreidimensionaler, vierdimensionaler oder fünfdimensionaler Code ist.

3. Die Methode gemäß einem der Ansprüche 1 oder 2, wobei der mehrdimensionale Code als weitere Dimension eine Farbcodierung über die Emissionsspektren der Photoluminenzfarbstoffe und/oder als weitere Dimension eine Farbcodierung über die Absorptionsfarbe der Farbstoffe enthält.

4. Die Methode gemäß einem der Ansprüche 1 bis 3, wobei die Tintenformulierungen separat nebeneinander und optional übereinander auf die Oberfläche des Produkts gedruckt werden, um den mehrdimensionalen Code zu generieren.

5. Die Methode gemäß einem der Ansprüche 1 bis 4, wobei sich die Photolumineszenzlebensdauern der Photolumineszenzfarbstoffe in den Tintenformulierungen in dem Bereich von 1 ns bis 1 min, vorzugsweise im Bereich von 1 ns bis 1 s, stärker bevorzugt im Bereich von 1 ns bis 1 ms, am stärksten bevorzugt im Bereich von 5 ns bis 100 µs unterscheiden.

6. Die Methode gemäß einem der Ansprüche 1 bis 5, die zusätzlich folgende Schritte enthält:
- Speichern des mehrdimensionalen Codes in mindestens einer Datenbank;
- Abfragen des mehrdimensionalen Codes aus der Datenbank und
- Abgleich dieses Codes mit dem detektierten Code zur Verifizierung oder Authentifizierung des Produkts.

7. Die Methode gemäß einem der Ansprüche 1 bis 6, wobei die Tintenformulierung zusätzlich auf mindestens eine Fläche der Oberfläche einer Verpackungsgruppe, die das Produkt enthält, beispielsweise ausgewählt aus Bündeln, Umverpackung, Paletten, in Form des mehrdimensionalen Codes und/oder auf Produktschilder (Tags), Barcode-Karten und Barcode-Etiketten in Form des mehrdimensionalen Codes aufgedruckt wird.

8. Die Methode gemäß einem der Ansprüche 1 bis 7, wobei der mehrdimensionale Code ein einzigartiger mehrdimensionaler Code ist, wobei vorzugsweise zur Generierung des einzigartigen mehrdimensionalen Codes mindestens eine Bezugsgröße des Produkts mithilfe eines einzigartigen Schlüssels verschlüsselt wird.

9. Die Methode gemäß einem der Ansprüche 1 bis 8, wobei während der Detektionsdauer zu festgelegten Zeitpunkten zwei oder mehrere Aufnahmen und/oder Spektren der emittierten Strahlung gemessen werden.

10. Die Methode gemäß einem der Ansprüche 1 bis 9, wobei die Photolumineszenzfarbstoffe Fluoreszenzfarbstoffe, die ausgewählt sind aus organischen Farbstoffen und/oder anorganischen Farbstoffen, bevorzugt halbleitende anorganische Nanokristalle, am stärksten bevorzugt halbleitenden anorganischen Nanokristalle ausgewählt aus der Gruppe der Perovskite, I-VI-Halbleiter, II-VI-Halbleiter, III-V-Halbleiter, IV-VI-Halbleiter, I-III-VI-Halbleiter, Carbon dots und Mischungen daraus, und/oder Phosphoreszenzfarbstoffe, die ausgewählt sind aus dotieren Oxiden, Nitriden, Oxynitriden, Sulfiden, Seleniden, Haliden, Silikaten und Aluminaten von Calcium, Strontium, Barium, Zink, Cadmium, Mangan, Silizium und Seltenerdmetallen und Mischungen daraus, bevorzugt aus dotierten Sulfiden und Aluminate von Calcium, Strontium, Barium und Zink, besonders bevorzugt aus mit Bismut dotierten Calcium/Strontiumsulfid, Kupfer dotiertem Zinksulfid, Europium dotiertem Strontiumaluminat, und/oder Anti-Stoke Farbstoffe enthalten.

11. Die Methode gemäß einem der Ansprüche 1 bis 10, wobei die emittierte Strahlung ein individuelles, zeitabhängiges Photolumineszenzspektrum ergibt, das mit einem Spektrometer detektiert wird.

12. Optisches, zeitabhängiges Sicherheitsmerkmal auf mindestens einer Fläche der Oberfläche eines Produktes in Form eines mehrdimensionalen Codes, das zwei oder mehrere Photolumineszenzfarbstoffe enthält, die unter Photonenanregung eine Strahlung im Bereich von 380-3000 nm emittieren und sich in ihren Photolumineszenzlebensdauern unterscheiden, wobei das optische, zeitabhängige Sicherheitsmerkmal mit der Methode nach einem der Ansprüche 1-11 auf mindestens einer Fläche der Oberfläche des Produktes aufgedruckt wird.

13. Verwendung des optischen, zeitabhängigen Sicherheitsmerkmals gemäß Anspruch 12 als cyber-physical system (CPS) zur Produktüberwachung und/oder zur Dokumentensicherung.

14. Serialisierungs- und/oder Track & Tracesystem, das ein optisches, zeitabhängiges Sicherheitsmerkmal enthält, das einen auf ein Produkt aufgedruckten mehrdimensionalen Code gemäß der Methode nach einem der Ansprüche 1-11 enthält.

15. Verwendung eines auf ein Produkt aufgedruckten mehrdimensionalen Codes gemäß der Methode nach einem der Ansprüche 1-11 als optisches, zeitabhängiges Sicherheitsmerkmal in einem Serialisierungs- und/oder Track & Tracesystem und/oder zur Dokumentensicherung.

## Claims

1. Method for labelling products, which method contains the following steps:
- providing ink formulations containing one or more photoluminescent dyes, preferably one photoluminescent dye, which, when subjected to photon excitation, emit radiation in the range of 380-3000 nm, preferably of 450 to 1800 nm, most highly preferably of 750 nm to 1100 nm;
- generating a multidimensional code for identifying a product, wherein at least one dimension, preferably two dimensions, is/are a spatial dimension;
- printing the ink formulations onto at least one area of the surface of the product in the form of this multidimensional code;
- irradiating the product printed with the ink formulation with photons;
- detecting the radiation emitted by the irradiated product in the range of 380 to 3000 nm, preferably of 450 to 1800 nm, most highly preferably of 750 nm to 1100 nm, wherein
two or more ink formulations are provided, all of which each contain one or more photoluminescent dyes, preferably one photoluminescent dye, wherein the ink formulations differ in terms of different photoluminescence lifetimes of the photoluminescent dyes;
one dimension of the multidimensional code is a temporal dimension, based on the photoluminescence lifetimes of the photoluminescent dyes;
**characterized in that**
the temporal progression of the radiation emitted by the irradiated product over a time period of 1 ns to 1 min, preferably 1 ns to 1 s, more highly preferably 1 ns to 1 ms, most highly preferably 5 ns to 100 µs, after the beginning of the irradiation is detected,
and **in that**
the proportion of the photoluminescent dyes in the ink formulations, independently of one another, is in each case 0.01% to 70.0% by weight, preferably 0.05% to 40.0% by weight, most highly preferably 0.09% to 30.0% by weight, measured relative to the total weight of the respective ink formulation.

2. Method according to Claim 1, wherein the multidimensional code is a three-dimensional, four-dimensional or five-dimensional code.

3. Method according to either of Claims 1 and 2, wherein the multidimensional code contains, as a further dimension, a colour coding relating to the emission spectra of the photoluminescent dyes and/or, as a further dimension, a colour coding relating to the absorption colour of the dyes.

4. Method according to any of Claims 1 to 3, wherein the ink formulations are printed separately next to one another and optionally one above another onto the surface of the product in order to generate the multidimensional code.

5. Method according to any of Claims 1 to 4, wherein the photoluminescence lifetimes of the photoluminescent dyes in the ink formulations differ in the range of 1 ns to 1 min, preferably in the range of 1 ns to 1 s, more highly preferably in the range of 1 ns to 1 ms, most highly preferably in the range of 5 ns to 100 µs.

6. Method according to any of Claims 1 to 5, which additionally contains the following steps:
- storing the multidimensional code in at least one database;
- retrieving the multidimensional code from the database and
- comparing this code with the detected code in order to verify or authenticate the product.

7. Method according to any of Claims 1 to 6, wherein the ink formulation is additionally printed onto at least one area of the surface of a packaging group containing the product, for example selected from bundles, external packaging, pallets, in the form of the multidimensional code and/or onto product tags, barcode cards and barcode labels in the form of the multidimensional code.

8. Method according to any of Claims 1 to 7, wherein the multidimensional code is a unique multidimensional code, wherein preferably for the purpose of generating the unique multidimensional code, at least one reference variable of the product is encrypted with the aid of a unique key.

9. Method according to any of Claims 1 to 8, wherein two or more recordings and/or spectra of the emitted radiation are measured at defined points in time during the detection duration.

10. Method according to any of Claims 1 to 9, wherein the photoluminescent dyes contain fluorescent dyes selected from organic dyes and/or inorganic dyes, preferably semiconducting inorganic nanocrystals, most highly preferably semiconducting inorganic nanocrystals selected from the group of the perovskites, I-VI semiconductors, II-VI semiconductors, III-V semiconductors, IV-VI semiconductors, I-III-VI semiconductors, carbon dots and mixtures thereof, and/or phosphorescent dyes selected from doped oxides, nitrides, oxynitrides, sulphides, selenides, halides, silicates and aluminates of calcium, strontium, barium, zinc, cadmium, manganese, silicon and rare earth metals and mixtures thereof, preferably from doped sulphides and aluminates of calcium, strontium, barium and zinc, particularly preferably from bismuth-doped calcium/strontium sulphide, copper-doped zinc sulphide, europium-doped strontium aluminate, and/or anti-Stokes dyes.

11. Method according to any of Claims 1 to 10, wherein the emitted radiation produces an individual, time-dependent photoluminescence spectrum, which is detected by a spectrometer.

12. Optical, time-dependent security feature on at least one area of the surface of a product in the form of a multidimensional code, which security feature contains two or more photoluminescent dyes which, when subjected to photon excitation, emit radiation in the range of 380-3000 nm and differ in their photoluminescence lifetimes, wherein the optical, time-dependent security feature is printed on at least one area of the surface of the product using the method according to any of Claims 1-11.

13. Use of the optical, time-dependent security feature according to Claim 12 as a cyber-physical system (CPS) for product monitoring and/or for document safeguarding.

14. Serialization and/or track & trace system containing an optical, time-dependent security feature containing a multidimensional code that has been printed onto a product in accordance with the method according to any of Claims 1-11.

15. Use of a multidimensional code that has been printed onto a product in accordance with the method according to any of Claims 1-11 as an optical, time-dependent security feature in a serialization and/or track & trace system and/or for document safeguarding.

## Revendications

1. Procédé d'étiquetage de produits qui comprend les étapes suivantes :
- fourniture de formulations d'encre contenant un ou plusieurs colorants photoluminescents, de préférence un colorant photoluminescent, qui émettent, sous photoexcitation, un rayonnement dans la plage de 380 à 3 000 nm, de préférence de 450 à 1 800 nm, le plus préférablement de 750 nm à 1 100 nm ;
- génération d'un code multidimensionnel pour l'identification d'un produit, au moins une dimension, de préférence deux dimensions, étant une/des dimension(s) locale(s) ;
- impression des formulations d'encre sur au moins une zone de la surface du produit sous la forme de ce code multidimensionnel ;
- irradiation, par des photons, du produit imprimé par la formulation d'encre ;
- détection du rayonnement émis par le produit irradié dans la plage de 380 à 3 000 nm, de préférence de 450 à 1 800 nm, le plus préférablement de 750 nm à 1 100 nm, dans lequel
au moins deux formulations d'encre sont fournies, qui contiennent chacune un ou plusieurs colorants photoluminescents, de préférence un colorant photoluminescent, les formulations d'encre se différenciant par différentes durées de vie de photoluminescence des colorants photoluminescents ;
une dimension du code multidimensionnel étant une dimension temporelle, basée sur la durée de vie de photoluminescence des colorants photoluminescents ;
**caractérisé en ce que**
la cinétique du rayonnement émis par le produit irradié est détectée pendant une durée de 1 ns à 1 min, de préférence de 1 ns à 1 s, plus préférablement de 1 ns à 1 ms, le plus préférablement de 5 ns à 100 µs après le début de l'irradiation,
et **en ce que**
la proportion de colorants photoluminescents dans les formulations d'encre indépendamment les uns des autres est de 0,01 à 70,0 % en poids, de préférence de 0,05 à 40,0 % en poids, le plus préférablement de 0,09 à 30,0 % en poids, mesurée par rapport au poids total de la formulation d'encre respective.

2. Procédé selon la revendication 1, le code multidimensionnel étant un code tridimensionnel, à quatre dimensions ou à cinq dimensions.

3. Procédé selon l'une quelconque des revendications 1 ou 2, dans lequel le code multidimensionnel contient comme dimension supplémentaire un codage couleur sur les spectres d'émission des colorants photoluminescents et/ou comme dimension supplémentaire un codage couleur sur la couleur d'absorption des colorants.

4. Procédé selon l'une quelconque des revendications 1 à 3, dans lequel les formulations d'encre sont imprimées séparément côte à côte et éventuellement les unes sur les autres sur la surface du produit afin de générer le code multidimensionnel.

5. Procédé selon l'une quelconque des revendications 1 à 4, dans lequel les durées de vie de photoluminescence des colorants photoluminescents dans les formulations d'encre se différencient dans la plage de 1 ns à 1 min, de préférence dans la plage de 1 ns à 1 s, plus préférablement dans la plage de 1 ns à 1 ms, le plus préférablement dans la plage de 5 ns à 100 µs.

6. Procédé selon l'une des revendications 1 à 5, qui contient en outre les étapes suivantes :
- stockage du code multidimensionnel dans au moins une base de données ;
- interrogation du code multidimensionnel à partir de la base de données et
- mise en correspondance de ce code avec le code détecté pour vérifier ou authentifier le produit.

7. Procédé selon l'une quelconque des revendications 1 à 6, la formulation d'encre étant en outre imprimée sur au moins une zone de la surface d'un groupe d'emballage qui contient le produit, par exemple choisi parmi des paquets, un suremballage, des palettes, sous la forme du code multidimensionnel et/ou sur des plaques signalétiques de produit (étiquettes), des cartes à code à barres et des étiquettes à code à barres sous la forme du code multidimensionnel.

8. Procédé selon l'une quelconque des revendications 1 à 7, dans lequel le code multidimensionnel est un code multidimensionnel unique, dans lequel, de préférence, pour générer le code multidimensionnel unique, au moins une donnée de référence du produit est cryptée au moyen d'une clé unique.

9. Procédé selon l'une quelconque des revendications 1 à 8, dans lequel deux ou plusieurs enregistrements et/ou spectres du rayonnement émis sont mesurés pendant la durée de détection à des instants spécifiés.

10. Procédé selon l'une quelconque des revendications 1 à 9, dans lequel les colorants fluorescents photoluminescents sont choisis parmi les colorants organiques et/ou les colorants inorganiques, de préférence les nanocristaux inorganiques semi-conducteurs, le plus préférablement les nanocristaux inorganiques semi-conducteurs choisis dans le groupe des pérovskites, des semi-conducteurs I-VI, des semi-conducteurs II-VI, des semi-conducteurs III-V, des semi-conducteurs IV-VI, des semi-conducteurs I-III-VI, des points de carbone et leurs mélanges, et/ou les colorants phosphorescents qui sont choisis parmi les oxydes, nitrures, oxynitrures, sulfures, séléniures, halogénures, silicates et aluminates, dopés, de calcium, strontium, baryum, zinc, cadmium, manganèse, silicium et métaux des terres rares et leurs mélanges, de préférence des sulfures et aluminates, dopés, de calcium, strontium, baryum et zinc, de manière particulièrement préférée parmi un sulfure de calcium/strontium dopé au bismuth, un sulfure de zinc dopé au cuivre, un aluminate de strontium dopé à l'europium et/ou des colorants anti-stokes.

11. Procédé selon l'une quelconque des revendications 1 à 10, dans lequel le rayonnement émis donne un spectre de photoluminescence individuel dépendant du temps, qui est détecté à l'aide d'un spectromètre.

12. Dispositif de sécurité optique dépendant du temps sur au moins une zone de la surface d'un produit sous la forme d'un code multidimensionnel qui contient deux colorants photoluminescents ou plus qui émettent un rayonnement dans la plage de 380 à 3 000 nm sous photoexcitation et se différencient par leur durée de vie photoluminescente, le dispositif de sécurité optique dépendant du temps étant imprimé sur au moins une zone de la surface du produit par le procédé selon l'une quelconque des revendications 1 à 11.

13. Utilisation du dispositif de sécurité optique dépendant du temps selon la revendication 12 comme système cyberphysique (CPS) pour la surveillance de produits et/ou pour la sauvegarde de documents.

14. Système de sérialisation et/ou de suivi et de traçage comprenant un dispositif de sécurité optique dépendant du temps contenant un code multidimensionnel imprimé sur un produit selon le procédé selon l'une quelconque des revendications 1 à 11.

15. Utilisation d'un code multidimensionnel imprimé sur un produit selon le procédé selon l'une quelconque des revendications 1 à 11 en tant que dispositif de sécurité optique dépendant du temps dans un système de sérialisation et/ou de suivi et de traçage et/ou pour la sauvegarde de documents.
